# EUROPEAN PATENT SPECIFICATION

(11) **EP 1 765 453 B1**
(45) Date of publication and mention of the grant of the patent: **23.11.2011**
(21) Application number: 05774570.5
(22) Date of filing: 15.07.2005
(51) Int. Cl.: A61B 17/00

(54) **Cannula for in utero surgery**
Kanüle für Operation in Gebärmutter
Canule pour operation in utero

(30) Priority: 15.07.2004 US 891937
(43) Date of publication of application: 28.03.2007
(73) Proprietor: Children's Medical Center Corporation, Boston, MA 02115 (US)
(72) Inventor: LOCK, James, Boston, MA 02115 (US); KIERCE, Paul, C., Woburn, MA 01801 (US); MARSHALL, Audrey, C., Boston, MA 02115-5737 (US)
(74) Representative: Austin, Hedley William
(86) International application number: PCT/US2005/025203
(87) International publication number: WO 2006/020055

(56) References cited:
- EP-A- 0 885 624
- EP-A- 1 072 280
- WO-A-00/33909
- US-A- 3 419 010
- US-A- 3 719 737
- US-A- 4 976 684
- US-A- 5 743 880
- US-A1- 2004 015 151

## Description

### BACKGROUND OF THE INVENTION

### 1. Technical Field

The present invention relates to a cannula for insertion into tissue during surgical procedures, in combination with a staightener for the cannula.

### 2. Description of the Related Art

There are many minimally invasive surgical procedures wherein surgery is performed without making a large incision in a patient Patients of these processes benefit by receiving less trauma to the body and save money by reduced hospitalization time and reduced therapy time. Such minimally invasive procedures range from cardiovascular, spinal, laparoscopic, thoracoscopic, and various anesthesia procedures. Minimally invasive surgery is also commonly known as endoscopic surgery because an endoscope is inserted to view the inside of the body so that the physicians can monitor the path of their instruments.

During a surgical procedure, a cannula mounted coaxially on a sharp-pointed trocar or blunt pointed obturator is commonly used to percutaneously access vessels and internal structures. The point on the trocar or obturator is used to puncture through surrounding structures and tissues to lead the cannula to the vessel or structure of interest. Once the tip of the trocar/obturator and the tip of the cannula are in the structure of interest, the trocar/obturator is removed and the cannula remains. The lumpen of the cannula, previously occupied by the trocar/obturator, can then be used to introduce or deliver various items such as pharmaceuticals, diagnostic or therapeutic devices, implantables, and instruments into the vessel or structure to perform the needed surgery or procedure.

In order to perform balloon dilation procedures in fetuses, one must be able to insert and remove a balloon dilation catheter at the structure of interest. These catheters are straight and blunt, with a very flexible shaft and an irregular profile, particularly after inflation/deflation. For this reason, the balloon is introduced and removed through an introducer cannula.

Foetal cardiac interventions, however, demand specific qualities of the trocar/obturator and introducer cannula. The combination of the trocar/obturator and cannula must be straight and inflexible through a length of 10-15 cm in order to have exact control of the tip of the trocar/obturator as it is advanced through maternal and foetal tissue to the target structure. Performing cardiac surgery on a foetus can require placement of wires or catheters at an angle different from the angle of straight access. The cannula must remain inflexible after the trocar/obturator has been removed to allow precise tip control.

There are many other procedures where, in order to gain access to a distant vascular or nonvascular chamber, the operation of an instrument needs to occur at a different angle than the angle of entry of the straight cannula/obturator combination. Therefore, there is a need for an inflexible cannula that can change the entry angle of an instrument at the surgical site of interest.

WO00/33909 discloses an infusion needle having a preformed bend that is straightened when inserted into an outer cannula for introduction to the target site. When the needle is deployed from the outer cannula (that is, when the outer cannula is retracted), the needle returns to its preformed configuration. The needle is used to aspirate or infuse material at multiple points by rotating the needle.

The present invention provides a combination which can deliver an instrument around a curve, such that once an introducer cannula is in position and curved, an instrument can be inserted through the cannula to follow the curve of the deployed cannula. This contrasts with the arrangement of WO00/33909 which can only deliver the needle in a straight direction, whereupon the needle curves once deployed when the outer cannula is retracted.

### SUMMARY OF THE INVENTION

According to the present invention there is provided a combination of an introducer cannula for insertion into tissue and a straightener, the introducer cannula including an elongated rigid hollow tube having a proximal end, a distal end, and a passageway extending therebetween. The distal end includes a memory of directionality to bend about a radius. The cannula straightener straightens the distal end of the cannula when inserted through the passageway of the cannula. The combination has the features defined in claim 1.

### BRIEF DESCRIPTION ON THE DRAWINGS

Other advantages of the present invention will be readily appreciated as the same becomes better understood by reference to the following detailed description when considered in connection with the accompanying drawings wherein:
Figure 1 is a side view of an introducer cannula with a curved distale end;
Figure 2 is a side view of an introducer cannula and straightener combination;
Figure 3 is a side view of one embodiment of a straightener,
Figure 4 is a cross-sectional view taken along line A in Figure 1 of the cannula showing a circular shape;
Figure 5 is a cross-sectionat view taken along line A in Figure 1 of the cannula showing an oblong shape;
Figure 6 is a cross-sectional view taken along line A in Figure 1 of the cannula showing a square shape;
Figure 7 is a partial view of a proximal end of a cannula showing an ergonomic knob;
Figure 8 is a side view of a proximal end of a cannula with an indicator,
Figure 9 is a perspective view of a proximal end of a cannula with an indicator;
Figure 10 is a perspective view of a proximal end of a cannula with an indicator showing alignment with the bend of the distal end;
Figure 11 is a side view of a proximal end of a cannula with an indicator of a light;
Figure 12 is a side view of a proximal end of a cannula with an indicator of a computer screen;
Figure 13 is a cross-sectional view taken along line B of Figure 1 of the shape of the distal end;
Figure 14 is a partial view of a distal end of a cannula showing a leading edge;
Figure 15 is a partial view of a straightener; with a pointed mercedes tip;
Figure 16 is a cross-setional view taken along line C of Figure 15 of the mercedes tip;
Figure 17 is a partial view of a straightener with a blunt tip;
Figure 18 is a cross-sectional view taken along line D of Figure 2 of a straightener filling the passageway of a cannula;
Figure 19 is a cross-sectional view taken along line D of Figure 2 of a straightener partially filling the passageway of a cannula;
Figure 20 is a cut-away view of a cannula with an instrument inside the passageway;
Figure 21 is a cut-away view of a cannula with an instrument inside the passageway where the functional end of the instrument is at a different angle than the proximal end of the instrument;
Figure 22 is a perspective view of a "D" shaped cross-section of a straightener;
Figure 23 is a perspective view of a "U" shaped cross-section of a straightener;
Figure 24 is a perspective view of an "I' shaped cross-section of a straightener;
Figure 25 is a side view of an alternative embodiment of the combination of the present invention;
Figure 26 is a side view of an alternative embodiment of the combination of the present invention.
Figure 27 is a side view of an alternative embodiment of the combination of the present invention
Figures 28A and 28B are side views of an alternative embodiment of the combination of the present invention;
Figures 29A and 29B are side views of an alternative embodiment of the combination of the present invention; and
Figures 30A and 30B are side views of an alternative embodiment of the combination of the present invention.

### DETAILED DESCRIPTION

Generally, the present invention provides a apparatus for use in performing minimally invasive surgery. More specifically, the present invention provides a cannula and cannula straightener combination for insertion into tissue. The cannula and cannula straightener combination is particularly useful in performing minimally invasive surgery where there is not easy access to a surgical site when inserting instruments in a straight direction.

An "introducer cannula" refers to a surgical tube inserted into a body cavity, duct, or tissue to drain fluid, deliver medication, or allow surgery to be performed at a remote site by inserting instruments through the cannula. An introducer cannula in this application is alternatively called just "a cannula"

A "cannu!a straightener" is an elongated solid or hollow rod for insertion in a cannula. A cannula straightener is also referred to as a "straightener," but can be called by others in various needed fields by various names. The straightener can be an obturator, a stylus, a trocar, or other similar device.

The term "tissue" means an aggregation of morphologically similar calls and associated intercellular matter acting together to perform one or more specific functions in the body. Four basic types of tissues include muscle, nerve, epidermal, and connective tissues. Tissue can refer to such specifics as vascular tissue, body cavity units, etc.

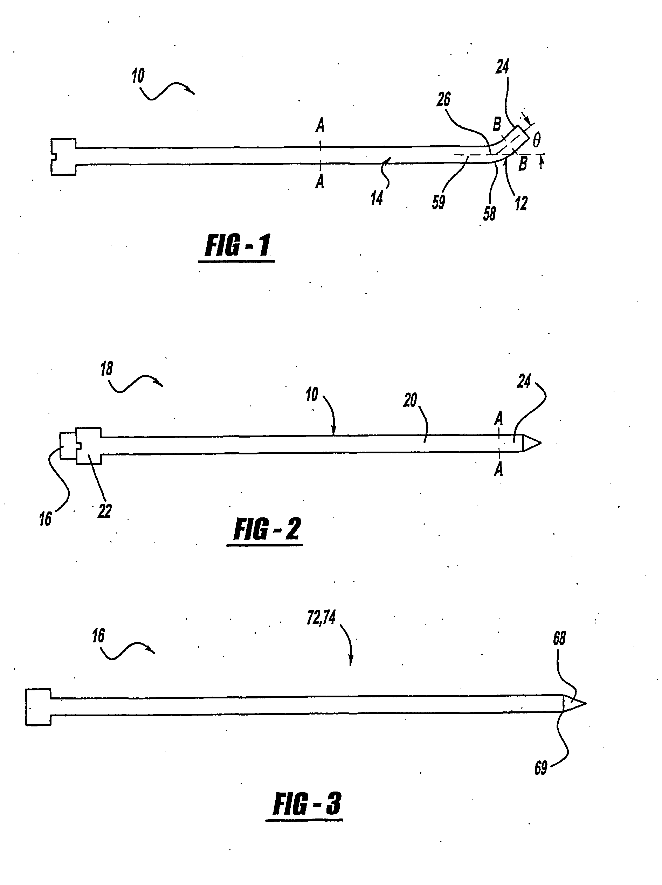

The cannula and cannula straightener combination of the present invention includes a cannula generally shown at 10 having a bendable arm 12 that maintains the structure and the functionality of a passageway 14 inside the cannula 10, as shown in Figure 1. The passageway 14 has a generally round cross-seational shape throughout its length. The functionality of the passageway 14 is to allow passage thereby of an insertion device or straightener or a medical instrument, as described below.

The cannula 10 is used with a cannula straightener 16. In Figure 2, a cannula and cannula straightener combination 18 for insertion in tissue includes the cannula 10 being an elongated rigid hollow tube 20 having a proximal end 22, a distal end 24, and the passageway 14 extending therebetween. The distal end 24 of the cannula. 10 includes a memory of directionality, as described below, to bend about a radius 26. A cannula straightener 16, shown alone in Figure 3, acts to straighten the distal end 24 of the cannula 10 when inserted through the passageway 14 of the cannula 10 as described below.

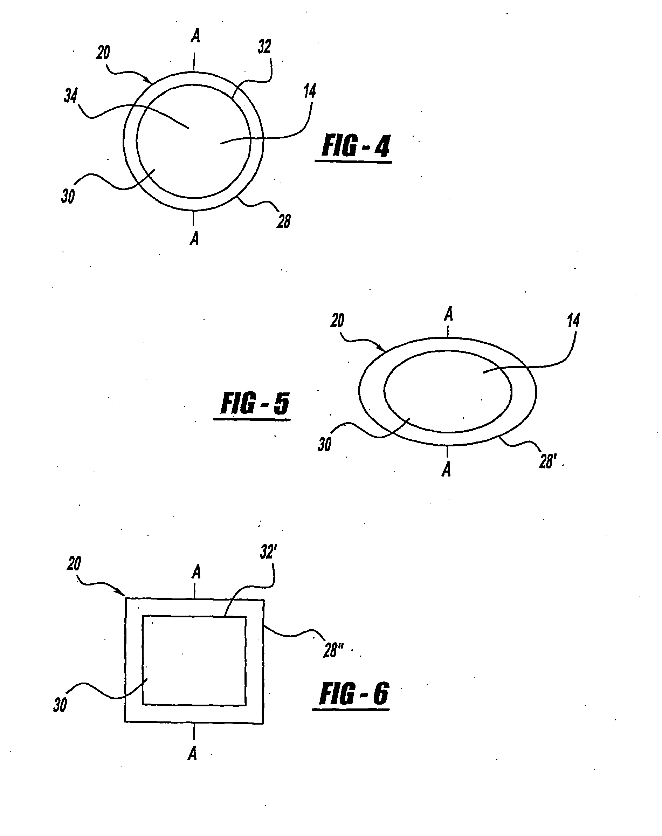

Preferably, the tube 20 has an outer circular cross-sectional shape 28 for smooth insertion into and through tissue. Other shapes can be used such as an oblong 28', square 28", or any other suitable shape as shown in Figures 4, 5, and 6.

The passageway 14 includes an inner surface 30 that can be any cross-sectional shape 32, such as circular 32 or square 32 as shown in Figures 4 and 6. The inner surface 30 can be custom designed to fit and guide a specific instrument 36 for insertion through the cannula 10. The inside diameter 34 of the passageway 14 can be any diameter appropriate for an instrument 36 to fit through in a surgical or other procedure. The cannula 10 can be of any suitable length to perform a specific procedure. The cannula 10 will likely be longer for use in fetal operations because it reaches through the tissue of the mother, through the womb, and through the fetal tissue to the surgical site. The cannula 10 can be ail one piece, or alternatively, the proximal end 22 and distal end 24 can be fixably attached to the cannula tube 20.

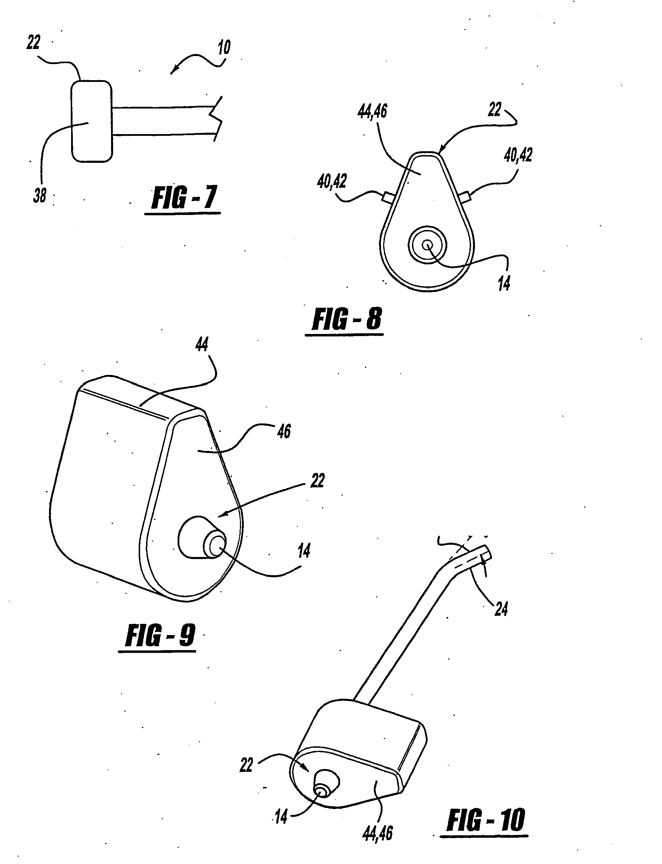

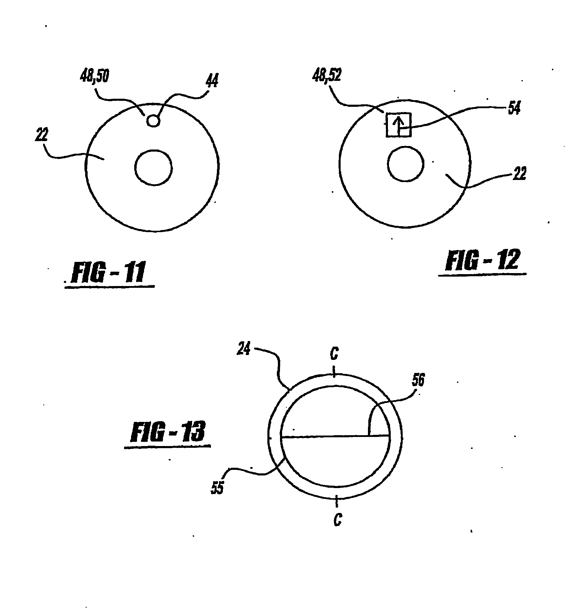

The proximal end 22 of the cannula 10, which is not inserted through tissue and will remain outside of the patient, can be any shape. For examples, as shown in Figure 7, the proximal end 22 can be an ergonomic knob 38 for the physician to hold onto during a surgical procedure. The proximal end 22 can also includes valves 40 and other ports 42 for liquid and/or gas entering the cannula 10, for irrigation and/or suction, for sample collection, or for pressure transduction as shown in Figure 8. Preferably, the proximal end 22 includes an indicator 44 for indicating the direction of the distal end 24 as shown in Figures 8, 9, and 10. The indicator 44 includes a signal 46 that allows the user to know the direction of the curve of the distal end 24. The indicator 44 allows for rotation of the cannula 10 to result in predictable redirection of its distal end 24.without angling or repositioning the entire cannula 10. As in Figures 8, 9, and 10, the indicator 44. includes elongated material in the direction of the bend 80 of the cannula 10. Alternatively, the direction of the distal end 24 can be indicated by an electronic device 48 such as a light 50 or on a computer screen 52, shown in Figures 11 and 12. For example, the indicator 44 could have an arrow 54 pointing in the direction of the bend 80. Any other suitable indicator 44 can be used.

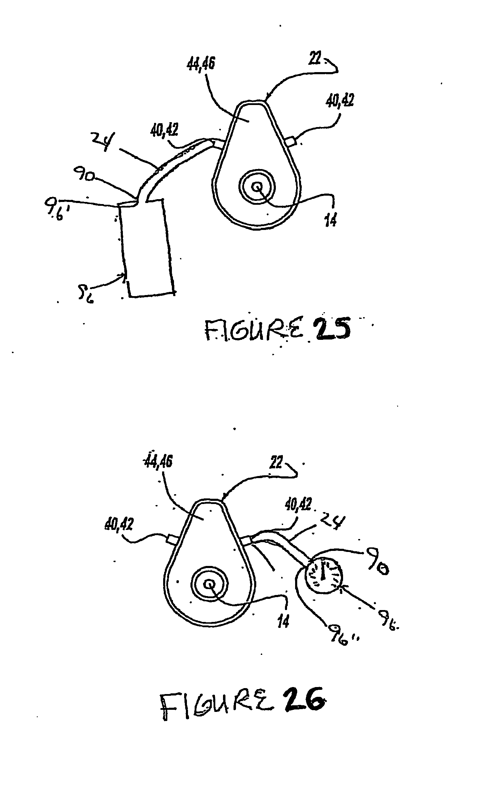

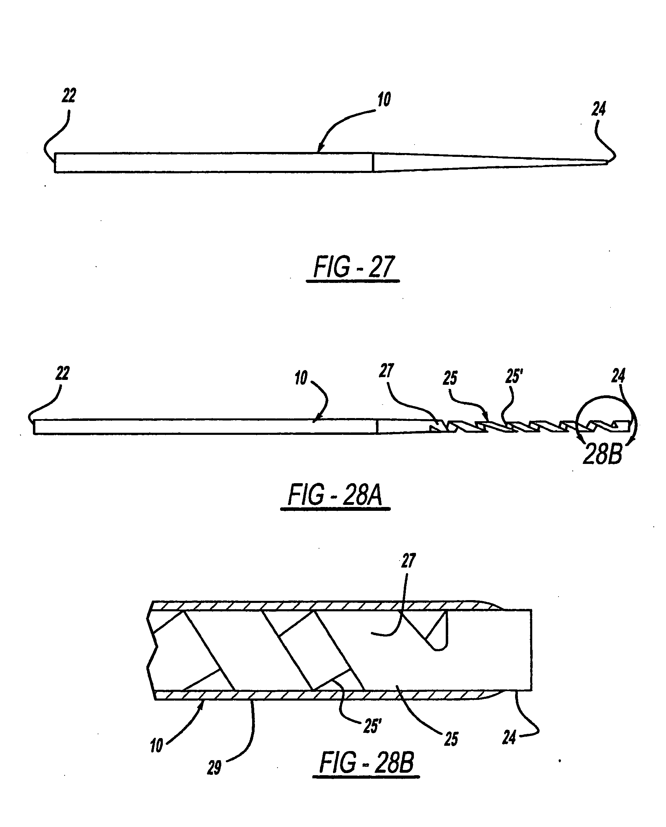

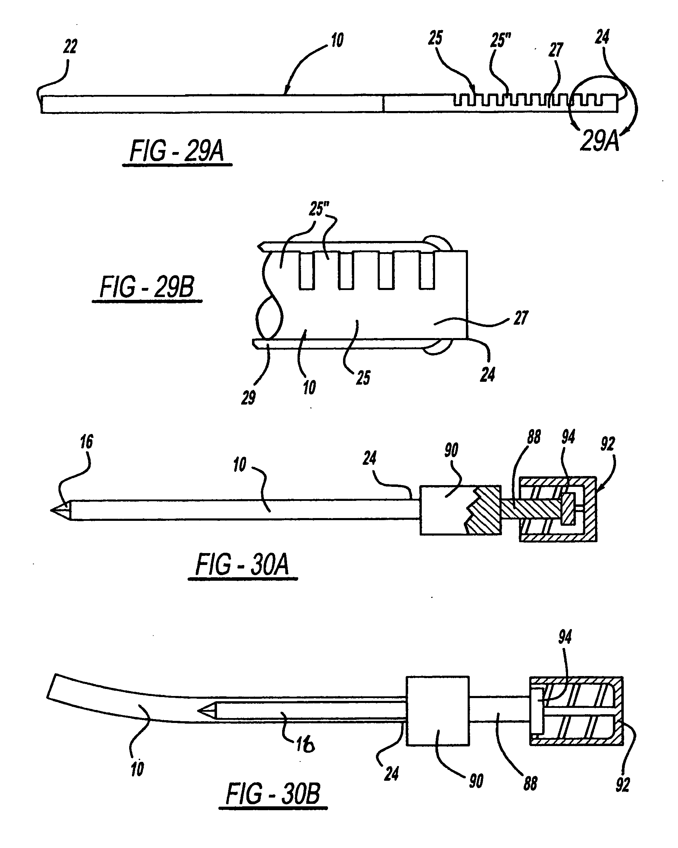

The distal end 24 of the cannula 10 is closest to the site of operation in the patient. The distal end 24 can be the same shape 55 and cross-sectional diameter 56 as the remainder of the cannula 10, shown in Figure 13, or alternatively, it can be wider or narrower. Additionally, the distal end 24 can be tapered as shown in Figure 25. The tapering limits grinding on the distal end 24 of the cannula 10 and the cannula 10 tapers as is approaches the distal end 24. The thickness of the cannula 10 can also be modified. For example, the cannula 10 at the proximal end 22 is preferably thicker than at the distal end 24. Specifically, the thickness at the proximal end 22 can be approximately 0.13mm (0.005 inches) and the thickness at the distal end 24 can be approximately 50 microns (0.002 inches). Further, the distal end 24 can include a texture portion 25, such as those shown in Figures 28A, 28B, 29A, and 29B. As shown in Figures 28A and 28B, the textured portion can include a spiral cut 25' on the exterior surface 27 of the distal end 24. After creating the spiral cut 25' on the distal end 24, the cannula 10 can be heat set for maintain the shape. Optionally, a tube 29 can be affixed about the exterior surface of the spirals 25' on the distal end 24 to increase the stretch resistance. The tube 29 can be any biocompatible material that can be affixed to the cannula 10, examples of such materials are known to those of skill in the art. The preferred material is heat shrinkable. A preferred compound is a plastic such as heat shrink polyethylene terephthalate (PET). The texture portion 25 on the distal end 24 can also include slots 25". The slots 25" can be sized to allowed easier flexibility of the distal end 24. A tube 29 can be affixing to the exterior surface of the slots 25" distal end 24 to increase the stretch resistance. The tube 29 can be any biocompatible material that can be affixed to the cannula. 10, examples of such materials are known to those of skill in the art. The preferred material is heat shrinkable. Preferably, the distal end 24 is about 3 to 4 mm in length, however, the distal end 24 can be any suitable length. The length can be made specific to a procedure. The distal end 24 can bend about a radius 26, from a memory of directionality, as shown in Figures 1 and 10. The distal end 24 bends at its juncture 58 with the cannula 10. The radius of bending can be any suitable angle theta, preferably 0 to 90 degrees from an axis 59 of the passageway 14, more preferably 0 to 60 degrees, and even more preferably 10 to 15 degrees from the axis 59 of the passageway 14. A memory of directionality allows the distal end 24 to return to the same angle theta each time a straightener 16 inside the cannula 10 is removed. The memory of directionality is a property of the material of the distal end 24.

During the bending of the distal end 24, the structural integrity and functionality of the passageway 14 in the cannula 10 is maintained. The cannula 10 does not kink so that an instrument 36 can fit through the passageway 14 without obstruction. The cannula 10 is rigid enough to withstand tissue pressure when inserted into and when inside a patient's tissue. The rigidness is maintained during rotation or maneuvering of the cannula 10 while in tissue, such as when maneuvering the distal end 24 after the straightener 16 is removed. The rigidness runs along the entire length of the cannula 10, including the distal end 24. Therefore, while the distal end 24 is flexible enough to bend around about a radius 26, it is also rigid enough to maintain the structural integrity and functionality of the passageway 14. This feature of the present invention is unlike many flexible plastic cannulas that kink and lose their passageway when a straightener is removed while inside tissue.

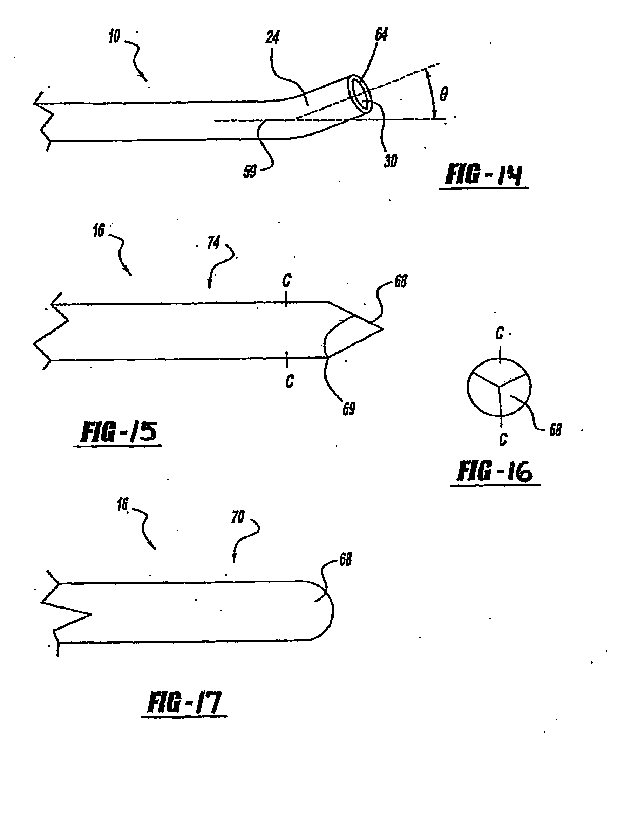

The distal end 24 further includes a leading edge 64 that is preferably tapered in toward inner surface 30 of the passageway 14, as shown in Figure 14. The tapering allows the cannula 10 to move through tissue more easily, especially when the cannula 10 is in combination with the straightener 16. The distal end 24 can also be blunt.

A shape memory material can be used for the cannula 10, such as a shape memory polymer or other shape memory materials such as Nitinol. Preferably, a rigid shape memory material is used. In general, a shape memory material undergoes a change of crystal structure at its transformation temperature. Superelasticity, or pseudo elasticity, occurs when a material is in an environment that is above the temperature of its transformation temperature. The lower temperature crystal structure can be formed by applying stress to the materials. Once sufficient stress is applied to the material above the transformation stress, the material undergoes deformation. Upon releasing the applied stress, the material returns to its original shape with no permanent deformation.

Preferably, the cannula 10 is made from Nitinol, which comes from a family of intermetallic materials that contain a nearly equal mixture of nickel (55 wt. %) and titanium. NITINOL is an acronym for Nicker Titanium Navel Ordnance Laboratory. Nitinol exhibits a unique phase transformation in the crystal structure when transitioning between the Austenite phase (high temperature, stronger state) and Martensite phase (low temperature, weaker state).

The behaviors shown in the phase transformation are commonly known as "Superelasticity' and "Shape Memory". Superelasticity occurs when nitinol is mechanically deformed at a temperature above its. Austenite Finish (At) temperature. This deformation causes a sbess-induced phase transformation from Austenite to Martensite. The stress-induced Martensite is unstable at temperatures above Af, and when the stress is removed, the material will immediately spring back to the Austenite phase and its pre-stressed position. Recoverable strains on the order of 8% are attainable. The high degree of elasticity, or "superelasticity", is the most attractive property of nitinol and the most common aspect of the material in use today.

Shape Memory occurs when the nitinol is in its Martensitic phase and is deformed to a new shape. When the material is then heated above the Af temperature, it changes back to Austenite and the deformation is lost as the material returns to its pre-deformed, original shape. Up to 8% shape recovery is possible. Nitinol first was marketed for its thermal shape memory properties as pipe couplings, connectors and actuators. All nitinol exhibits both superelastic and shape memory behavior, but alloy composition and the material's thermo-mechanical processing history dictate the temperatures where these properties exist

Any other material exhibiting shape memory behaviour can also be used. For example, thermoplastic polymers can be used. A thermoplastic polymer can have one shape at room temperature, and transform into another shape at body temperature. The cannula 10 can also be made from other materials, such as a combination of plastic and metal, in other words, a combination of metallic and non-metallic materials. For example, the cannula 10 can be made of a stainless steel braid with a Teflon outer jacket.

It is also desirable that the cannula 10 be imagable during an operation. The cannula 10 can be made with an imagable material so that the location of the cannula 10 in the patient's body can be determined by imaging methods such as ultrasound, magnetic resonance imaging (MRI), computed tomography (CT), X-ray, fluoroscopy, nuclear imaging or any other imaging method known in the art. In order for a cannula 10 to be imagable in an X-ray visualization procedure, the cannula 10 must be more absorptive of the X-rays than the surrounding tissues. Radiopaque materials are commonly used such as stainless steel and nickel-titanium alloys. Radiopaque markers can also be used. In MRI, polymers are typically used. Any other suitable imaging material can be used. The cannula 10 can be made of a combination of imagable materials and other biocompatible and/or shape memory materials. Methods of manufacturing the cannula 10 from the materials above are well known in the art.

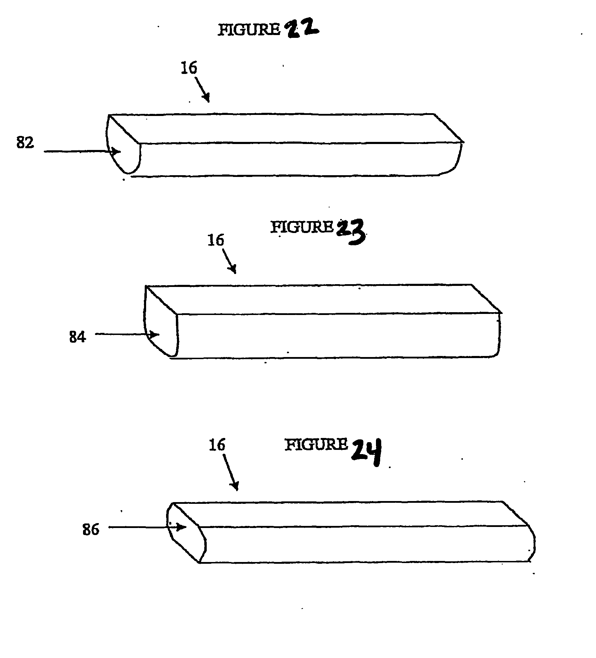

The cannula straightener 16 straightens the distal end 24 of the cannula 10 when inserted through the passageway 14 of the cannula 10. In general, the straightener 16 is a straight elongated rod as shown in Figure 3. The straightener 16 can also be any other suitable mechanism or device capable of straightening the cannula 10. The straightener 16 can take on a variety of cross-sectional shapes as shown in Figures 22, 23, and 24 such as a "D" shape 82, a "U" shape 84, or an "I" shape 86. The "D" shape is essentially a round rod with a flat portion. The "U" shape is essentially a square rod with rounded corners on one side. The "I" shape is essentially a round rod with flat portions on opposite sides. These shapes aid in preventing the pushing of debris back into the patient when the straightener 16 is inserted into the cannula 10. The straightener 16 can include a tip 68 on a distal end 69. The tip 68 can be any umber of shapes. For example, the tip 68 can be pointed as shown in Figures 3 and 15. More specifically, Figures 15 and 16 shows a tip 68 with a pointed Mercedes tip 68. A pointed tip 68 is useful in making a sharp and less obtrusive puncture in tissue. The tip 68 can also be blunt, as shown in Figure 17. Preferably, the tip 68 extends less than 1 mm beyond the leading edge 64 of the distal end 24 of the cannula 10. A short length of the tip 68 is desired because when the cannula and straightener combination 18 is positioned at the site of interest, and the straightener 16 is removed, the distal end 24 should not be far from the site. The straightener 16 can be hollow or solid.

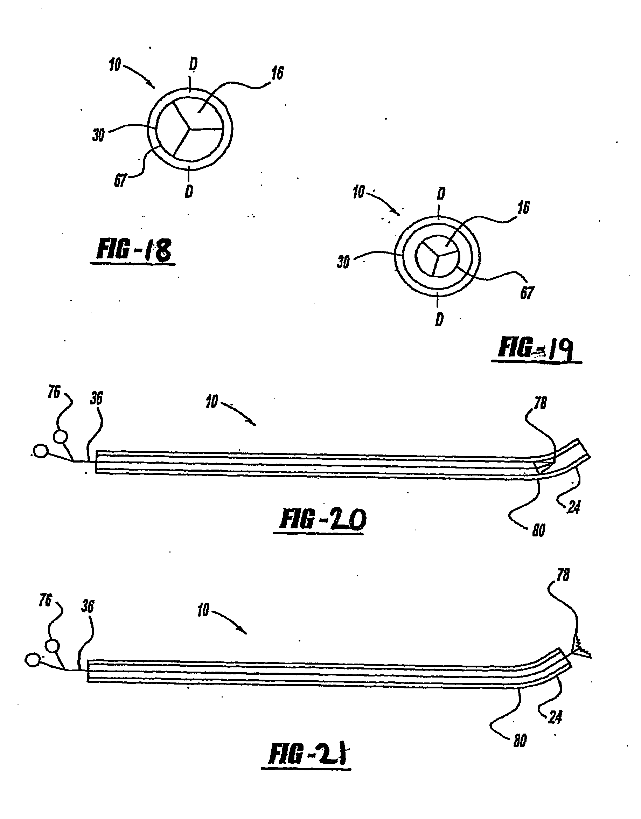

The straightener 16 can completely fill the passageway 14 as shown in Figure 18. In other words, an outside surface 67 of the straightener 16 can be removably integral with the inner surface 30 of the passageway 14. A completely filled passageway 14 is ideal when it is desirable for no bodily fluids to escape up the passageway 14. Alternatively, as shown in Figure 19, the outside surface 67 of the straightener 16 can be a distance from the inner surface 30 of the passageway so that the straightener 16 only partially fills the passageway 14.

The straightener 16 can be made from any suitable material. For example, the straightener 16 can be made of plastic or mental. The straightener 16 can be made of a material capable of being autoclave for reuse in multiple procedures. Alternatively, the straightener 16 can be made of a disposable material. Methods of manufacturing the straightener 16 are well known in the art. The straightener 16 can be imagable in the same way as the cannula 10 as described above.

The straightener 16 can take on various forms and functions. For example, the straightener can be an obturator 70. An obturator 70 generally has a blunt tip 68, and is used when damage to surrounding delicate surface tissues is to be minimized. The straightener 16 can also be a stylus 72. The straightener 16 can also be a trocar 74. A trocar 74 generally includes a pointed tip 68 for puncturing tissue. The tip 68 of the straightener 16 can be distinguishable from the cannula 10 by the imaging methods described above.

In order to reduced friction between the cannula 10 and the straightener 16 either the surface of the cannula 10 or the straightener 16 can be made of a lubricious, non-galling material. One example of such a material includes, but is not limited to, nodular, thin, dense chrome (NTDC). Alternatively, as shown in Figures 30A and 30B, a junction 88 can be formed between a hub 90 on the proximal end 24 of the cannula 10 and a hub 92 on the proximal end 94 of the straightener 16. Preferably, the junction 88 is threaded thereby enabling more controlled motion between the cannula 10 and the straightener 16. The junction 88 is long enough to completely withdraw the straightener 16 through the cannula bend.

The cannula 10 can also include at least one attachment 96 that can be attached via a hub 90 on the proximal end 24 of the cannula 10 as shown in Figures, 25 and 26. The attachment 96 can be any device capable of being affixed to the hub 90 of the cannula 10. Examples of such attachments 96 include, but are not limited to, a collection container 96' and a manometer 96".

As stated above, the attachment 96 can be a collection container 96'. The container 96' is affixed to the hub 90 of the cannula 10. The container 96' can include an aspirator, if necessary, for the removal of material via the cannula 10. The container 96' enables samples to be collected from the individual in which the cannula 10 is placed. The sample is obtained through the passageway 14 of the cannula 10 using the distal end 24 of the cannula; In other words, the distal end 24 of the cannula 10 can be manipulated such that the distal end 24 is in contact or close proximity with the sample material. The sample material can then be extracted through the passageway 14 of the cannula 10 and collected in the container 96'.

Alternatively, the attachment 96 can be a manometer 96", which is a device which measures pressure. The manometer 96" can measure pressure wherever the distal end 24 of the cannula 10 is located.

In use, an introducer cannula 10 is inserted into a patient's tissue by inserting the straight tube 20 through tissue and curving a length of the tube at the bendable arm 12 when at a site of operation. This insertion is further defined by inserting the introducer cannula and cannula straightener combination 18 as described above into the tissue in a straight direction. The cannula 10 can be guided to the site of interest. The cannula 10 can further be guided by use of the indicator 44 on the proximal end 22 to indicate in which direction the cannula distal end 24 will curve. Insertion in a straight direction occurs because the distal end 24 of the cannula 10 is straightened by being in combination with the straightener 16. It would be very difficult or impossible to insert and guide the cannula 10 to the site of interest if the distal end 24 were curved. When in combination 18, the cannula 10 and the straightener 16 are straight in order to have exact control over the tip of the straightener.

When the cannula 10 is at the site of interest, a length of the cannula 10 is curved. The distal end 24 of the cannula 10 is bent about a radius 26. The bending of the distal end 24 can be accomplished by any of the methods as described above. The distal end 24 can be bent about any suitable radius 26 at any angle theta as described above. During the bending of the distal end 24, the passageway 14 is maintained inside the cannula 10. When the straightener 16 is a straight elongated rod 66 such as an obturator 70, stylus 72, or trocar 74, the straightener 16 is removed from the cannula 10 to allow the distal end 24. to return to its curved position. During removal of the straightener 16, the cannula 10 maintains the passageway 14 through its length.

Once the distal end 24 is curved at the site of interest, it can be slightly adjusted again by using the indicator 44. Adjustment should only be fine adjusting so as not to tear any adjacent tissues or structures.

An instrument 36 can be inserted through the passageway 14 as shown in Figures 20 and 21. Any instrument 36 can be inserted for the surgery or procedure of interest Such instruments 36 are manipulated by a physician at a proximal end 76, and are functional at a distal end 78.The instrument 36 is able to curve around the bend 80 of the distal end 24 of the cannula 10, and therefore at least a portion of the instrument 36 is flexible. The functional end 78 of the instrument 36 is able to perform the desired function while curved about the radius 26 of the distal end 24. When the desired procedure is finished, the instrument 36 is removed from the cannula 10. During removal, the instrument distal end 78 curves back through the distal end 24 of the cannula 10. This process of inserting an instrument 36 and removing can be repeated to perform different procedures through the cannula 10.

The cannula 10 Is removed when the procedure is finished. The cannula 10 can be removed as it is, i.e, in the curved distal end 24 position. The cannula 10 can be removed by straightening the distal end 24 of the cannula 10 about the radius 26 and removing the cannula 10 from tissue. The cannula 10 can also be removed by inserting a cannula straightener 16 as described above into the cannula 10, straightening the distal end 24 of the cannula. 10 about a radius 26, and removing the cannula and cannula straightener combination 18 from tissue.

Surgery can be performed by inserting an introducer cannula 10 into tissue by any method as described above, introducing an instrument 36 through a passageway 14 of the cannula 10 while maintaining the bend 80 of the distal end 24 of the cannula 10 as described above, utilizing the instrument 36 to perform at least one step of a surgical procedure, removing the instrument 36 from the surgical site and from the cannula 10, and removing the introducer cannula 10 from the tissue by any method as described above.

There are many procedures of interest where it is desirable that a cannula 10 be inserted straight through tissue but then be able to change the directionality of the distal end 24 of the cannula 10 where an operative procedure is taking place. A curved distal end 24 of the cannula 10 is useful in reaching tissues and structures unreachable from a straight insertion of a cannula 10. The cannula 10 can also be used to manipulate the tissue. For example, in fetal uses, the cannula 10 (or several cannulae) can be inserted such that the distal end 24 of the cannula 10 is in contact with fetal tissue. The distal end 24 can then be manipulated in order to effectuate a change in position of the fetus within the uterus. The distal end 24 of the cannula can be advanced, redirected, and spun in order to effectuate the movement of the fetus or other tissue in need of such movement.

For example, the cannula 10 can be used in operative procedures on a fetus. In this procedure, the introducer cannula and straightener combination 18 is inserted through the tissue of the mother (abdominal wall, navel intravaginally), through the womb, and through the fetal tissue to the site of interest as described in the above methods. The distal end 24 of the cannula 10 is curved about a radius 26, and the passageway 14 is maintained according to the methods described above. An instrument 36 can be inserted in the passageway 14 to perform a surgical procedure of the operation. After removal of the instrument 36, another instrument 36 can be inserted or another step of the procedure can commence. The introducer cannula 10 can then be removed from the fetal tissue, womb, and mother's tissue by the methods described above.

The cannula 10 is also useful for biliary cannulation in a transhepatic approach. Cannulation can be performed with small tapered catheters designed to guide wires or injections of contrast medium into biliary ducts. The need for biliary cannulation often occurs when there is an acute obstruction of the bile ducts, especially in patients with cholangitis. The obstruction can be a stone that has migrated down from the gallbladder. Patients with sepsis also can require drainage of the biliary tree. This can be accomplished by inserting a cannula 10 in a transhepatic approach.

The cannula 10 is also useful four fetal aortic valvuloplasty. Neonatal aortic stenosis, narrowing of the aortal valve, is a serious, though treatable, congenital heart condition. Several different procedures are used in treating neonatal aortic stenosis, such as percutaneous, transvascular balloon valvutoplasty, in which the aortic valve orifice is dilated using a balloon catheter. When aortic stenosis presents in the second trimester fetus, it can develop into hypoplastic left heart syndrome, a condition that is fatal if untreated. Treatment with aortic valvuloplasty in the fetus may be advantageous. The cannula 10 can be used to place a balloon in the aortic valve during fetal balloon valvuloplasty.

The cannula 10 is also useful for placing catheters into the brachial plexus for pain management Often, injuries to the brachial plexus cause pain that can be debilitating for many years. Regional anesthesia can be used during an operation instead of general anesthesia. Catheters can be placed in the brachial plexus to make a continuous nerve block to manage acute pain. The cannula 10 can be used to introduce catheters into the brachial plexus.

The cannula 10 is also useful for placing catheters into the epidural space for anesthesia. An epidural catheter can be placed through the skin into the epidural space of the spine by using the cannula 10. Catheters allow access to the epidural space for the administration of medication such as anesthetics. The catheters can be placed in the epidural space temporarily.

The cannula 10 is also useful in treating thoracic aortic dissection. Thoracic aortic dissection is one of the most common traumas to the aorta. The essential feature is a tear in the intimal layer of the aorta, followed by formation and propagation of a subintimal hematoma. Several diseases affect the media of the aorta and make it prone to dissection, such as Marfan, Ehlers-Danlos, and other connective tissue diseases, and pulsatile flow and high blood pressure can contribute to the propagation of the dissection. The cannula 10 can aid in placing a graft on the damaged aorta, in replacing a defective valve in the aorta, or in any other surgical procedure needed in the aorta.

The cannula 10 is further useful in laparoscopic dissection of tissue. Laparoscopic surgery is performed in the abdominal and pelvic regions. The cannula 10 can be used to introduce instruments 36. needed in the laparoscopic procedure such as a grasper or scissors at an angle to reach the surgical site.

The cannula 10 is also useful in hydro dissection procedures of laparoscopic surgery. Hydro dissection uses the force of pulsatile irrigation with crystalloid solutions to separate tissue planes. The operating field is kept clear during the procedure. Hydro dissection is currently used in pelvic lymhadenectomy and pleurectomy. The cannula 10 can be used to introduce a hydro dissection sprayer at a certain angle to an operation site.

In any of these procedures, it is desirable to image the cannula 10 during the placement and removal, and also during the operative procedure itself. The cannula can be guided to and from the operative site by using an imaging method such as ultrasound, MRI, CT, X-ray, fluoroscopy, or nuclear imaging. Any other suitable imaging method can also be used.

## Claims

1. In combination, an introducer cannula (10) including an elongate rigid hollow tube (20) having a proximal end (22), a distal end (24), and an elongate passageway (14) extending within the tube between said distal end and said proximal end, said distal end being of a material having a memory of directionality such that said distal end can be selectively constrained in a straightened condition or allowed to revert to an unconstrained bent condition in which said distal end is bent about a radius (26), at a predetermined acute angle **(O)** to said passageway, said passageway maintaining its structure and functionality when said distal end is in the straightened and bent conditions; and
straightening means (16) for straightening the introducer cannula, the straightening means comprising a straight shaft arranged for insertion along the length of said passageway so as to constrain said distal end in said straightened condition and for complete withdrawal along the length of said passageway via said proximal end, said withdrawal allowing said distal end to revert to said bent condition, said shaft having a tip (68) arranged to extend beyond said distal end when said distal end is constrained in said straightened condition.

2. A combination according to claim 1, wherein said tip is arranged to extend beyond said distal end by less than 1 mm.

3. A combination according to claim 1 or 2, wherein said distal end has a tapered leading edge (64).

4. A combination according to any of claims 1 to 3, wherein said proximal end includes indicator means (44) for indicating the direction of bend of said distal end in said bent condition.

5. A combination according to any of claims 1 to 4, wherein an outside surface of said cannula straightening means is shaped and dimensioned to completely fill said passageway.

6. A combination according to any of claims 1 to 5, wherein said straightening means comprises an obturator (70), stylus (72) or trocar (74).

7. A combination according to any of claims 1 to 5, which further comprises an instrument (36) for performing a surgical operation, said instrument being arranged to extend along said passageway and having a functional end (78) arranged to slide around said distal end in said bent condition and function outside of said passageway at a site of operation.

## Patentansprüche

1. Einführkanüle (10), einschließlich eines länglichen, steifen, hohlen Rohrs (20), das ein proximales Ende (22), ein distales Ende (24) und einen länglichen Durchgang (14) aufweist, der sich in dem Rohr zwischen dem distalen Ende und dem proximalen Ende erstreckt, wobei das distale Ende aus einem Material besteht, das derart ein Richtungsgedächtnis aufweist, dass das distale Ende selektiv in einen gestreckten Zustand gezwungen werden kann oder ihm ermöglicht wird, sich wieder in einen nicht-erzwungenen gebogenen Zustand zu begeben, bei dem das distale Ende um einen Radius (26) in einem im Voraus bestimmten spitzen Winkel (Θ) zu dem Durchgang gebogen ist, wobei der Durchgang seine Struktur und Funktionalität bewahrt, wenn das distale Ende sich in dem gestreckten und in dem gebogenen Zustand befindet; und Streckungsmittel (16) zum Strecken der Einführkanüle, wobei das Streckungsmittel einen gestreckten Schaft aufweist, der derart zum Einführen entlang der Länge des Durchgangs angeordnet ist, um das distale Ende in den gestreckten Zustand zu zwingen, und zum vollständigen Zurückziehen entlang der Länge des Durchgangs über das proximale Ende, wobei es das Zurückziehen ermöglicht, dass sich das distale Ende wieder in den gebogenen Zustand begibt, wobei der Schaft eine Spitze (68) aufweist, die angeordnet ist, um sich über das distale Ende hinaus zu erstrecken, wenn das distale Ende in die gestreckten Zustand gezwungen ist, in Kombination.

2. Kombination nach Anspruch 1, wobei die Spitze angeordnet ist, um sich weniger als 1 mm über das distale Ende hinaus zu erstrecken.

3. Kombination nach Anspruch 1 oder 2, wobei das distale Ende eine sich verjüngende Anfangskante (64) aufweist.

4. Kombination nach einem der Ansprüche 1 bis 3, wobei das proximale Ende Indikatormittel (44) zum Anzeigen der Richtung der Biegung des distalen Endes in dem gebogenen Zustand enthält.

5. Kombination nach einem der Ansprüche 1 bis 4, wobei eine Außenfläche des Kanülenstreckungsmittels geformt und dimensioniert ist, um den Durchgang vollständig zu füllen.

6. Kombination nach einem der Ansprüche 1 bis 5, wobei das Streckungsmittel einen Verschluss (Obturator) (70), einen Stift (72) oder einen Trokar (74) aufweist.

7. Kombination nach einem der Ansprüche 1 bis 5, welche des Weiteren ein Instrument (36) zum Durchführen einer chirurgischen Operation aufweist, wobei das Instrument angeordnet ist, um sich entlang des Durchgangs zu erstrecken, und ein funktionales Ende (78) aufweist, das angeordnet ist, um um das distale Ende in dem gebogenen Zustand zu gleiten und an der Operationsstelle außerhalb des Durchgangs zu funktionieren.

## Revendications

1. Combinaison d'une canule d'introduction (10) comprenant un tube creux rigide allongé (20) ayant une extrémité proximale (22), une extrémité distale (24), et un passage allongé (14) s'étendant dans le tube entre ladite extrémité distale et ladite extrémité proximale, ladite extrémité distale étant d'un matériau ayant une mémoire de directionnalité afin que ladite extrémité distale puisse être sélectivement contrainte dans un état raidi ou autorisée à revenir dans un état incurvé sans contrainte dans lequel ladite extrémité distale est incurvée autour d'un rayon (26), à un angle aigu prédéterminé (Θ) par rapport au dit passage, ledit passage maintenant sa structure et sa fonctionnalité lorsque ladite extrémité distale est dans l'état raidi ou dans l'état incurvé ; et
d'un moyen de raidissement (16) pour raidir la canule d'introduction, le moyen de raidissement comprenant une tige droite agencée pour être insérée dans la longueur dudit passage afin de contraindre ladite extrémité distale dans ledit état raidi et pour être retirée complètement de la longueur dudit passage par l'intermédiaire de ladite extrémité proximale, ledit retrait permettant à ladite extrémité distale de revenir à l'état incurvé, ladite tige ayant un embout (68) agencé pour s'étendre au-delà de ladite extrémité distale lorsque ladite extrémité distale est contrainte dans ledit état raidi.

2. Combinaison selon la revendication 1, dans laquelle ledit embout est agencé pour s'étendre au-delà de ladite extrémité distale de moins de 1 mm.

3. Combinaison selon la revendication 1 ou 2, dans laquelle ladite extrémité distale a un bord avant effilé (64).

4. Combinaison selon l'une quelconque des revendications 1 à 3, dans laquelle ladite extrémité proximale comprend un moyen d'indication (44) pour indiquer la direction de courbure de ladite extrémité distale dans ledit état incurvé.

5. Combinaison selon l'une quelconque des revendications 1 à 4, dans laquelle une surface extérieure dudit moyen de raidissement de canule est façonnée et dimensionnée pour remplir complètement ledit passage.

6. Combinaison selon l'une quelconque des revendications 1 à 5. dans laquelle ledit moyen de raidissement comprend un obturateur (70), un stylet (72) ou un trocart (74).

7. Combinaison selon l'une quelconque des revendications 1 à 5, comprenant en outre un instrument (36) pour effectuer une opération chirurgicale, ledit instrument étant agencé pour s'étendre le long dudit passage et ayant une extrémité fonctionnelle (78) agencée pour coulisser autour de ladite extrémité distale dans ledit état incurvé et fonctionner à l'extérieur dudit passage à un site d'opération.
